(19) Europäisches Patentamt
European Patent Office
Office européen des brevets

(11) EP 1 871 791 B1

(12) **EUROPEAN PATENT SPECIFICATION**

(45) Date of publication and mention of the grant of the patent:
**07.03.2012 Bulletin 2012/10**

(21) Application number: **06843735.9**

(22) Date of filing: **27.12.2006**

(51) Int Cl.:
***C07K 14/395*** (2006.01) ***C12N 1/19*** (2006.01)
***C12C 1/00*** (2006.01) ***C12G 1/00*** (2006.01)

(86) International application number:
**PCT/JP2006/326364**

(87) International publication number:
**WO 2007/099694 (07.09.2007 Gazette 2007/36)**

(54) **GENE ENCODING PROTEIN RESPONSIBLE FOR FLOCCULATION PROPERTY OF YEAST AND USE THEREOF**

GEN, DAS DAS FÜR DIE FLOCKUNGSEIGENSCHAFTEN VON HEFE VERANTWORTLICHE PROTEIN KODIERT, UND SEINE VERWENDUNG

GÈNE CODANT POUR UNE PROTÉINE RESPONSABLE DES PROPRIÉTÉS DE FLOCULATION D'UNE LEVURE ET APPLICATIONS

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.02.2006 JP 2006049223**

(43) Date of publication of application:
**02.01.2008 Bulletin 2008/01**

(73) Proprietor: **Suntory Holdings Limited**
**Kita-ku, Osaka-shi,**
**Osaka 530-8203 (JP)**

(72) Inventors:
- **NAKAO, Yoshihiro**
  **Mishima-gun, Osaka 618-8503 (JP)**
- **KODAMA, Yukiko**
  **Mishima-gun, Osaka 618-8503 (JP)**
- **SHIMONAGA, Tomoko**
  **Mishima-gun, Osaka 618-8503 (JP)**

(74) Representative: **Vossius & Partner**
**Siebertstrasse 4**
**81675 München (DE)**

(56) References cited:

- **BOWEN SUZANNE ET AL: "Patterns of polymorphism and divergence in stress-related yeast proteins" YEAST, vol. 22, no. 8, June 2005 (2005-06), pages 659-668, XP002431945 ISSN: 0749-503X**
- **DATABASE EPO Proteins [Online] 2 July 2002 (2002-07-02), "Sequence 2 from Patent EP1209235." XP002431947 retrieved from EBI accession no. EPOP:AX441343 Database accession no. AX441343 & EP 1 209 235 A (NAT INST OF ADVANCED IND SCIEN [JP]) 29 May 2002 (2002-05-29)**
- **VERSTREPEN K J ET AL: "Yeast flocculation: What brewers should know" APPLIED MICROBIOLOGY AND BIOTECHNOLOGY, SPRINGER VERLAG, BERLIN, DE, vol. 61, no. 3, May 2003 (2003-05), pages 197-205, XP002426016 ISSN: 0175-7598**
- **KLIS FRANS M ET AL: "DYNAMICS OF CELL WALL STRUCTURE IN SACCHAROMYCES CEREVISIAE" FEMS MICROBIOLOGY REVIEWS, ELSEVIER, AMSTERDAM, NL, vol. 26, no. 3, August 2002 (2002-08), pages 239-256, XP009080890 ISSN: 0168-6445**


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 871 791 B1

Printed by Jouve, 75001 PARIS (FR)

SEQUENCE LISTING

<110> Suntory Limited

<120> Gene encoding protein responsible for flocculation property of yeast and use thereof

<130> PCT06-0134

<150> JP2006-49223
<151> 2006-02-24

<160> 6

<170> PatentIn version 3.3

<210> 1
<211> 1221
<212> DNA
<213> Saccharomyces sp.

<400> 1

```
atgcaatata aaaagacttt ggtcgcctct gctttggccg ctactacatt ggccgcttat      60
gctccatccg agccatggtc cactttgact ccaacagcta cttacagcgg aggtattacc     120
gactacgttt ccactttcgg tattgccgtt caaccaatct ccaccacatc ttctgccgct     180
gccacagcct cctccaaggc taagagagct gcttcccaaa tcggtgacgg ccaaatccaa     240
gccgccacta ccaccgctgc tgtctccacc aagactactg ccccagctgt ttctcaaatt     300
ggtgacggtc aaatccaagc taccaccaag actactgccg ctgctgtctc ccaaatcggt     360
gatggtcaaa tccaagccac caccaagact acctcagcca agaccactgc cgctgctgtt     420
tctcaaattg gcgacggtca aatccaagct accacaaaga ccacttcagc taagactact     480
gccgctgccg tttctcaaat tggtgatggt caaattcaag ccaccaccaa gaccactgcc     540
gctgctgttt ctcaaattgg cgacggtcaa atccaagcta ccacaaagac cacttcagct     600
aagactactg ccgctgctgt ctcccaaatc ggtgatggtc aaattcaagc tactaccact     660
actttagctc caaagagtac cgctgctgcc gtttctcaaa tcggtgacgg tcaaatccaa     720
gccaccacaa ctacttcagc caagtctagc gccgctgctg tatctcaaat cactgacggt     780
caagtccaag ctaccaccaa aacctctact actcaagctg ccagccaagt aagtgacggc     840
caagtccaag ctaccaccgg tactgctacc tccacctccg ctgaagcttc ttctacctct     900
actgacccag tcgatgctgt ttcctgtaag aactcgggta ctttggaaat gaacttgaag     960
gaaggtatct taactgacgg taagggcaga atcggttcca ttgttgccaa cagacaattc    1020
```

caattcgatg gtccaccacc acaagccggt gccatctacg ctgccggttg gtccatcacc 1080
ccagatggta acttagctat cggtgacaac gatgtcttct accaatgttt gtctggtact 1140
ttctacaact tgtacgacga acacattggt actcaatgta ctccagtcca cttagaagct 1200
attgatttga tagactgtta a 1221

<210> 2
<211> 406
<212> PRT
<213> Saccharomyces sp.

<400> 2

Met Gln Tyr Lys Lys Thr Leu Val Ala Ser Ala Leu Ala Ala Thr Thr
1 5 10 15
Leu Ala Ala Tyr Ala Pro Ser Glu Pro Trp Ser Thr Leu Thr Pro Thr
20 25 30
Ala Thr Tyr Ser Gly Gly Ile Thr Asp Tyr Val Ser Thr Phe Gly Ile
35 40 45
Ala Val Gln Pro Ile Ser Thr Thr Ser Ser Ala Ala Ala Thr Ala Ser
50 55 60
Ser Lys Ala Lys Arg Ala Ala Ser Gln Ile Gly Asp Gly Gln Ile Gln
65 70 75 80
Ala Ala Thr Thr Thr Ala Ala Val Ser Thr Lys Thr Thr Ala Pro Ala
85 90 95
Val Ser Gln Ile Gly Asp Gly Gln Ile Gln Ala Thr Thr Lys Thr Thr
100 105 110
Ala Ala Ala Val Ser Gln Ile Gly Asp Gly Gln Ile Gln Ala Thr Thr
115 120 125
Lys Thr Thr Ser Ala Lys Thr Thr Ala Ala Ala Val Ser Gln Ile Gly
130 135 140
Asp Gly Gln Ile Gln Ala Thr Thr Lys Thr Thr Ser Ala Lys Thr Thr
145 150 155 160
Ala Ala Ala Val Ser Gln Ile Gly Asp Gly Gln Ile Gln Ala Thr Thr
165 170 175
Lys Thr Thr Ala Ala Ala Val Ser Gln Ile Gly Asp Gly Gln Ile Gln
180 185 190
Ala Thr Thr Lys Thr Thr Ser Ala Lys Thr Thr Ala Ala Ala Val Ser
195 200 205
Gln Ile Gly Asp Gly Gln Ile Gln Ala Thr Thr Thr Thr Leu Ala Pro
210 215 220

Lys Ser Thr Ala Ala Ala Val Ser Gln Ile Gly Asp Gly Gln Ile Gln
225 230 235 240
Ala Thr Thr Thr Thr Ser Ala Lys Ser Ser Ala Ala Ala Val Ser Gln
245 250 255
Ile Thr Asp Gly Gln Val Gln Ala Thr Thr Lys Thr Ser Thr Thr Gln
260 265 270
Ala Ala Ser Gln Val Ser Asp Gly Gln Val Gln Ala Thr Thr Gly Thr
275 280 285
Ala Thr Ser Thr Ser Ala Glu Ala Ser Ser Thr Ser Thr Asp Pro Val
290 295 300
Asp Ala Val Ser Cys Lys Asn Ser Gly Thr Leu Glu Met Asn Leu Lys
305 310 315 320
Glu Gly Ile Leu Thr Asp Gly Lys Gly Arg Ile Gly Ser Ile Val Ala
325 330 335
Asn Arg Gln Phe Gln Phe Asp Gly Pro Pro Pro Gln Ala Gly Ala Ile
340 345 350
Tyr Ala Ala Gly Trp Ser Ile Thr Pro Asp Gly Asn Leu Ala Ile Gly
355 360 365
Asp Asn Asp Val Phe Tyr Gln Cys Leu Ser Gly Thr Phe Tyr Asn Leu
370 375 380
Tyr Asp Glu His Ile Gly Thr Gln Cys Thr Pro Val His Leu Glu Ala
385 390 395 400
Ile Asp Leu Ile Asp Cys
405

<210> 3
<211> 40
<212> DNA
<213> Artificial

<220>
<223> Primer

<400> 3
gagctcatag cggccatgca atataaaaag actttggtcg 40

<210> 4
<211> 42
<212> DNA
<213> Artificial

<220>
<223> Primer

<400> 4

ggatcctatg cggccgcatg gatgcgacta gagcagtggt ag 42

<210> 5
<211> 70
<212> DNA
<213> Artificial Sequence

<220>
<223> Primer

<400> 5

aaagataatc atattcctct tcctacacta ataaaacaat aacaataaaa ccttgacagt 60
cttgacgtgc 70

<210> 6
<211> 70
<212> DNA
<213> Artificial Sequence

<220>
<223> Primer

<400> 6

ctaatcaatg caaaaaaaaa aacatcagaa ttgaattgat cgtttttcgt cgcacttaac 60
ttcgcatctg 70

## Description

### TECHNICAL FIELD

The present invention relates to a protein responsible for flocculation property of yeast and use thereof, in particular, a brewery yeast for producing alcoholic beverages with appropriate flocculation property and a method for producing said beverages. More particularly, the present invention relates to a yeast, which shows appropriate flocculation property by controlling expression level of non-ScHSP150 protein specific to a lager brewing yeast, to a method for selecting said yeast, and to a method for producing alcoholic beverages with said yeast.

### BACKGROUND ART

Flocculation property of yeast used for fermentation in alcoholic beverages is very important. Flocculation property of yeast means property of forming aggregation as a result of interaction among each individual yeast cells, thus sedimenting to the bottom of the liquid in a liquid culture medium.

For example, yeasts used for fermentation of lager-type beer, which is popularly drunk at the present day, are also called bottom fermenting yeast, because the yeast have a character of flocculating and sedimenting to the bottom of fermentation broth near the end of fermentation. In beer brewing, yeast is recovered after fermentation and the recovered yeast is used at the subsequent fermentation, which is called "Renjo" which means successive fermentation. Thus, flocculation property of yeast is very important property from the standpoint of working efficiency of brewing process. That is, yeast having poor flocculation property does not sediment at the end of fermentation, and there is a problem that extraneous steps such as centrifugation are required for recover it. On the other hand, yeast having undesirably high flocculation property may sediment during fermentation, which can result in immature termination of fermentation. In that case, flavor and taste of resultant product are also seriously influenced. Accordingly, it is very important to use yeast having suitable flocculation property for production of desired alcoholic beverages.

Further, flocculation of yeast is actively studied, because efficiency is required especially in the field of industrial alcohol production (Novel agglutinative alcohol fermenting yeast, Japanese Examined Patent Publication (Kokoku) No. H6-36734) and wastewater treatment (Japanese Patent No. 3044284), as well as in the field of alcoholic beverage production.

FLO gene family (FLO1, FLO5, FLO8, FLO9, FLO10, FLO11) and SFL1, etc. have ever been recognized as genes responsible for flocculation property of yeast as a result of enormous quantity of investigations on flocculation property, which is of importance for industry, of yeast as stated above.

In the investigation, for example, property of FLO1 gene was analyzed at molecular level (Bidard et al., YEAST, 11(9), 809, 1995). Method for controlling flocculation property of beer yeast was investigated as well using Flo1p (Japanese Patent No. 3643404).

Method of controlling flocculation property of yeast by regulating expression of FLO8 gene, and method of judging flocculating property using nucleotide sequence of FLO5 gene (International Publication No. WO01/040514), etc. are also reported.

On the other hand, some of yeast cell surface proteins specific to flocculating yeast are known as well. However, knowledge of each proteins is not sufficient for research to control flocculation property of beer yeast.

### DISCLOSURE OF INVENTION

Under the above situations, there has been a need for breeding yeasts having flocculation property suitable for production of desired alcoholic beverages to make high-efficiency production of alcoholic beverages possible by using a gene encoding a protein responsible for flocculation property of brewery yeast and said protein.

The present inventors made expensive studies to solve the above problems and as a result, succeeded in identifying and isolating a gene encoding a protein responsible for flocculation property of yeast from beer yeast. Moreover, the present inventors produced transformed yeast in which expression of the obtained gene was controlled to verify that flocculation property can be actually controlled, thereby completing the present invention.

Thus, the present invention relates to a protein encoded by the gene disclosed herein responsible for flocculation property of brewery yeast, to a transformed yeast in which the expression of said gene is controlled, to a method for controlling flocculation property using a yeast in which the expression of said gene is controlled, or the like. More specifically, disclosed are the following polynucleotides, a vector or a DNA fragment comprising said polynucleotide. The present invention provides a transformed yeast introduced with said vector or DNA fragment, a method for producing alcoholic beverages by using said transformed yeast, and the like.

Disclosed is:

(1) A polynucleotide selected from the group consisting of

(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 in which one to 10 amino acids thereof are deleted, substituted, inserted and/or added, and having an activity of imparting flocculation property to yeast;
(d) a polynucleotide comprising a polynucleotide encoding a protein having an amino acid sequence having 95% or higher identity with the amino acid sequence of SEQ ID NO:2, and said protein having an activity of imparting flocculation property to yeast;
(e) a polynucleotide comprising a polynucleotide which hybridizes to a polynucleotide consisting of a nucleotide sequence complementary to the nucleotide sequence of SEQ ID NO:1 under stringent conditions, and which encodes a protein having an activity of imparting flocculation property to yeast; and
(f) a polynucleotide comprising a polynucleotide which hybridizes to a polynucleotide consisting of a nucleotide sequence complementary to the nucleotide sequence of the polynucleotide encoding the protein having the amino acid sequence of SEQ ID NO:2 under stringent conditions, and which encodes a protein having an activity of imparting flocculation property to yeast.

(2) The polynucleotide according to (1) above selected from the group consisting of:

(g) a polynucleotide comprising a polynucleotide encoding a protein consisting of the amino acid sequence of SEQ ID NO: 2, or encoding the amino acid sequence of SEQ ID NO: 2 in which 1 to 5 amino acids thereof are deleted, substituted, inserted, and/or added, and wherein said protein has an activity of imparting flocculation property to yeast;
(h) a polynucleotide comprising a polynucleotide encoding a protein having 98% or higher identity with the amino acid sequence of SEQ ID NO: 2, and having an activity of imparting flocculation property to yeast; and
(i) a polynucleotide comprising a polynucleotide which hybridizes to a polynucleotide consisting of a nucleotide sequence of SEQ ID NO: 1 or which hybridizes to a polynucleotide' consisting of a nucleotide sequence complementary to the nucleotide sequence of SEQ ID NO: 1, under high stringent conditions, which encodes a protein having an activity of imparting flocculation property to yeast.

(3) The polynucleotide according to (1) above comprising a polynucleotide consisting of the nucleotide sequence of SEQ ID NO: 1.
(4) The polynucleotide according to (1) above comprising a polynucleotide encoding a protein consisting of the amino acid sequence of SEQ ID NO: 2.
(5) The polynucleotide according to any one of (1) to (4) above, wherein the polynucleotide is DNA.
(6) A polynucleotide selected from the group consisting of:

(j) a polynucleotide encoding RNA having a nucleotide sequence complementary to a transcript of the polynucleotide (DNA) according to (5) above;
(k) a polynucleotide encoding RNA that represses the expression of the polynucleotide (DNA) according to (5) above through an RNAi effect;
(l) a polynucleotide encoding RNA having an activity of specifically cleaving a transcript of the polynucleotide (DNA) according to (5) above; and
(m) a polynucleotide encoding RNA that represses the expression of the polynucleotide (DNA) according to (5) above through a co-suppression effect.

The present invention relates to:

(8) A vector containing the polynucleotide according to any one of (1) to (5) above.
(8a) The vector of (8) above, which comprises the expression cassette comprising the following components:
(x) a promoter that can be transcribed in a yeast cell;
(y) any of the polynucleotides described in (1) to (5) above linked to the promoter in a sense or antisense direction; and
(z) a signal that can function in a yeast with respect to transcription termination and polyadenylation of a RNA molecule.
(8b) The vector of (8) above, which comprises the expression cassette comprising the following components:
(x) a promoter that can be transcribed in a yeast cell;

(y) any of the polynucleotides described in (1) to (5) above linked to the promoter in a sense direction; and
(z) a signal that can function in a yeast with respect to transcription termination and polyadenylation of a RNA molecule.
(8c) The vector of (8) above, which comprises the expression cassette comprising the following components:
(x) a promoter that can be transcribed in a yeast cell;
(y) any of the polynucleotides described in (1) to (5) above linked to the promoter in a antisense direction; and
(z) a signal that can function in a yeast with respect to transcription termination and polyadenylation of a RNA molecule.
(9) A vector containing the polynucleotide according to (6) above.
(10) A protein encoded by the polynucleotide according to any one of (1)(a) to (d), (2)(g) and (h) and (3) to (5) above.
(11) A yeast into which a vector containing a polynucleotide as defined in (1)(a) to (d), (2)(g) and (h) and to (5) above has been introduced, wherein the flocculation property is increased.
(12) The yeast according to (11) above, wherein the flocculation property is increased by increasing an expression level of the protein of (10) above.
(13) A yeast, wherein the expression of the polynucleotide (DNA) according to (5) above is repressed by:

disrupting the gene corresponding to the polynucleotide (DNA) of (5) above.

(14) The yeast according to (13) above, wherein the flocculation property is decreased.
(15) A method for producing an alcoholic beverage by using the yeast according to any one of (11) to (14) above.
(16) The method according to (15) above, wherein the brewed alcoholic beverage is a malt beverage.
(17) The method according to (15) above, wherein the brewed alcoholic beverage is wine. Also disclosed is:
(18) An alcoholic beverage produced by the method according to any one of (15) to (17) above. The present invention also relates to:
(19) A method for assessing a test yeast for its flocculation property, comprising using a primer or probe designed based on the nucleotide sequence of a gene having the nucleotide sequence of SEQ ID NO: 1 and encoding a protein having an activity of imparting flocculation property to yeast.

Also disclosed is:

(19a) A method for selecting a yeast having a high or low flocculation property by using the method in (19) above.
(19b) A method for producing an alcoholic beverage (for example, beer) by using the yeast selected with the method in (19a) above.

The present invention also relates to:

(20) A method for assessing a test yeast for its flocculation property, comprising: culturing the test yeast; and measuring the expression level of the gene having the nucleotide sequence of SEQ ID NO: 1 and encoding a protein having an activity of imparting flocculation property to yeast.

Also disclosed is:

(20a) A method for selecting a yeast, which comprises assessing a test yeast by the method described in (20) above and selecting a yeast having a high or low expression level of gene encoding a protein having an activity of imparting flocculation property to yeast.
(20b) A method for producing an alcoholic beverage (for examples, beer) by using the yeast selected with the method in (20a) above.

Finally, the present invention relates to:

(21) A method for selecting a yeast, comprising: culturing test yeasts; quantifying the protein of (10) above or measuring the expression level of the gene having the nucleotide sequence of SEQ ID NO: 1 and encoding a protein having an activity of imparting flocculation property to yeast; and selecting a test yeast having an amount of the protein or the gene expression level according to the flocculation property of interest.
(22) The method for selecting a yeast according to (21) above, comprising: culturing a reference yeast and test yeasts; measuring for each yeast the expression level of the gene having the nucleotide sequence of SEQ ID NO: 1 and encoding a protein having an activity of imparting flocculation property to yeast; and selecting a test yeast having the gene expression higher or lower than that in the reference yeast.

(23) The method for selecting a yeast according to (21) above, comprising: culturing a reference yeast and test yeasts; quantifying the protein according to (10) above in each yeast; and selecting a test yeast having a larger or smaller amount of the protein than that in the reference yeast. That is, the method for selecting a yeast of (21) above, comprising: culturing plural yeasts; quantifying the protein of (10) above in each yeast; and selecting a yeast having a larger or smaller amount of the protein among them.
(24) A method for producing an alcoholic beverage comprising: conducting fermentation for producing an alcoholic beverage using the yeast according to any one of (11) to (14) above.

According to the method for producing alcoholic beverages using transformed yeast of the present invention, alcoholic beverages can be produced highly-efficiently by using yeasts having flocculation property suitable for production of desired alcoholic beverages.

## BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract concentration (w/w%).
Figure 3 shows the expression profile of non-ScHSP150 gene in yeasts upon beer fermentation test. The horizontal axis represents fermentation time while the vertical axis represents the intensity of detected signal.
Figure 4 shows the result of flocculation property test of non-ScHSP150 highly expressed strain. The vertical axis represents Segmentation Index indicating flocculation property.
Figure 5 shows the result of flocculation property test of non-ScHSP150 disrupted strain. The vertical axis represents Segmentation Index indicating flocculation property.

## BEST MODES FOR CARRYING OUT THE INVENTION

The present inventors isolated and identified non-ScHSP150 gene encoding a protein responsible for flocculation property of brewery yeast based on the lager brewing yeast genome information mapped according to the method disclosed in Japanese Patent Application Laid-Open No. 2004-383169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present disclosure provides (a) a polynucleotide comprising a polynucleotide of the nucleotide sequence of SEQ ID NO:1; and (b) a polynucleotide comprising a polynucleotide encoding a protein of the amino acid sequence of SEQ ID NO:2. The polynucleotide can be DNA or RNA.
The target polynucleotide is not limited to the polynucleotide encoding a protein responsible for flocculation property derived from lager brewing yeast described above and may includes other polynucleotides encoding proteins having equivalent functions to said protein. Proteins with equivalent functions include, for example, (c) a protein of an amino acid sequence of SEQ ID NO:2 with 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and imparting flocculation property to yeast. In general, the number of deletions, substitutions, insertions, and/or additions is preferably smaller. In addition, such proteins include (d) a protein having an amino acid sequence with about 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO:2, and imparting flocculation property to yeast In general, the percentage identity is preferably higher.
Flocculation property of yeast may be measured, for example, by a method described in Japanese Patent Application Laid-Open No. H8-205890.
Furthermore, also described is (e) a polynucleotide comprising a polynucleotide which hybridizes to a polynucleotide consisting of a nucleotide sequence complementary to the nucleotide sequence of SEQ ID NO: 1 under stringent conditions and which encodes a protein imparting flocculation property to yeast; and (f) a polynucleotide comprising a polynucleotide which hybridizes to a polynucleotide complementary to a nucleotide sequence of encoding a protein of SEQ ID NO:2 under stringent conditions, and which encodes a protein imparting flocculation property to yeast.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO:1 or polynucleotide encoding the amino acid sequence of SEQ ID NO:2 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO:2 as calculated by homology search software, such as PASTA and BLAST using default parameters.

Identity between amino acid sequences or nucleotide sequences may be determined using algorithm BLAST by Karlih and Altschul (Proc. Natl. Acad Sci. USA, 87: 2264-2268, 1990; Proc. Natl. Acad. Sci. US4, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

The polynucleotide may include (j) a polynucleotide encoding RNA having a nucleotide sequence complementary to a transcript of the polynucleotide (DNA) according to (5) above; (k) a polynucleotide encoding RNA that represses the expression of the polynucleotide (DNA) according to (5) above through RNAi effect; (L) a polynucleotide encoding RNA having an activity of specifically cleaving a transcript of the polynucleotide (DNA) according to (5) above; and (m) a polynucleotide encoding RNA that represses expression of the polynucleotide (DNA) according to (5) above through co-supression effect. These polynucleotides may be incorporated into a vector, which can be introduced into a cell for transformation to repress the expression of the polynucleotides (DNA) of (a) to (i) above. Thus, these polynucleotides may suitably be used when repression of the expression of the above DNA is preferable.

The phrase "polynucleotide encoding RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347, 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucleotide encoding RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed. RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof. Methods for producing or using such double-stranded RNA (dsRNA, siRNA or shRNA) are known from many publications (see, e.g., Japanese National Phase PCT Laid-open Patent Publication No. 2002-516062; US 2002/086356A; Nature Genetics, 24(2), 180-183,2000 Feb.; Genesis, 26(4), 240-244, 2000 April; Nature, 407:6802, 319-20, 2002 Sep. 21; Genes & Dev., Vol. 16, (8), 948-958, 2002 Apr.15; Proc. Natl. Acad. Sci. USA., 99(8), 5515-5520, 2002 Apr. 16; Science, 296(5567), 550-553, 2002 Apr. 19; Proc Natl. Acad. Sci. USA, 99:9, 6047-6052, 2002 Apr. 30; Nature Biotechnology, Vol.20 (5), 497-500, 2002 May; Nature Biotechnology, Vol. 20(5), 500-505, 2002 May; Nucleic Acids Res., 30:10, e46, 2002 May 15).

The phrase "polynucleotide encoding RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; Nucl. Acids. Res. 19: 6751, 1991; Protein Eng 3: 733, 1990; Nucl. Acids Res. 19: 3875, 1991; Nucl. Acids Res. 19: 5125, 1991; Biochem Biophys Res Commun 186: 1271, 1992). In addition, the phrase "polynucleotide encoding RNA that represses DNA expression through co-supression effect" refers to a nucleotide that inhibits functions of target DNA by "co-supression".

The term "co-supression" as used herein, refers to a phenomenon where when a gene having a sequence identical or similar to a target endogenous gene is transformed into a cell, the expressions of both the introduced foreign gene and the target endogenous gene are repressed. Design of polynucleotides having a co-supression effect can also be found in various publications (see, e.g., Smyth DR: Curr. Biol. 7: R793, 1997, Martienssen R: Curr. Biol, 6: 810, 1996).

## 2. Protein of the present invention

The present invention also provides proteins encoded by any of the polynucleotides (a) to (d), g) and (h) above. A preferred protein of the present invention comprises an amino acid sequence of SEQ ID NO:2 with one to 10 amino acids thereof being deleted, substituted, inserted and/or added, and imparts flocculation property to yeast.

Such protein includes those having an amino acid sequence of SEQ ID NO:2 with amino acid residues thereof of the number mentioned above being deleted, substituted, inserted and/or added and imparting flocculation property to yeast. In addition, such protein includes those having homology as described above with the amino acid sequence of SEQ ID NO:2 and imparting flocculation property to yeast.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad Sci. USA 79: 6409 (1982), Gene 34: 315 (1985), Nuc. Acids. Res., 13: 4431 (1985), Proc. Natl. Acad Sci. USA 82: 488 (1985).

Deletion, substitution, insertion and/or addition of one to 10 acid residues in an amino acid sequence of the protein of the invention means that one to 10 acid residues are deleted, substituted, inserted and/or added at any one to 10 positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

## 3. Vector and yeast transformed with the vector

The present disclosure then provides a vector comprising the polynucleotide described above. The vector may be a vector including any of the polynucleotides described in (a) to (i) above or any of the polynucleotides described in (j) to (m) above. Generally, the vector may comprise an expression cassette including as components (x) a promoter that can transcribe in a yeast cell; (y) a polynucleotide described in any of (a) to (i) above that is linked to the promoter in sense or antisense direction; and (z) a signal that functions in the yeast with respect to transcription termination and polyadenylation of RNA molecule.

According to the present invention, in order to highly express the protein of the invention described above upon brewing alcoholic beverages (e.g., beer) described below, these polynucleotides are introduced in the sense direction to the promoter to promote expression of the polynucleotide (DNA) as defined in the claims. Further, in order to repress the above protein of the invention upon brewing alcoholic beverages (e.g., beer) described below, these polynucleotides are introduced in the antisense direction to the promoter to repress the expression of the polynucleotide (DNA) as defined in the claims.

In order to repress the above protein of the invention, the polynucleotide may be introduced into vectors such that the polynucleotide of any of the (j) to (m) is to be expressed. According to the present invention, the target gene (DNA) may be disrupted to repress the expression of the DNA described above or the expression of the protein described above. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951 (1979), Methods in Enzymology, 101, 202(1983), Japanese Patent Application Laid-Open No.6-253826).

Further, in the present invention, the expression level of a target gene can be controlled by introducing a mutation to a promoter or genetically altering a promoter by homologous recombination. Such mutation introducing method is described in Nucleic Acids Res. 29, 4238-4250 (2001), and such alteration of a promoter is described in, for example, Appl Environ Microbiol., 72, 5266-5273 (2006).

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery, yeasts for beer, wine and sake. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad Sci. USA, 75: 1929(1978)), a lithium acetate method (J. Bacteriology, 153: 163 (1983)), and methods described *in* Proc. Natl. Acad Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 μg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

## 4. Method of producing alcoholic beverages according to the present invention and alcoholic beverages produced by the method

A yeast having flocculation property suitable for a target alcoholic beverages can be obtained by introducing the vector of the present disclosure or DNA fragments described above to a yeast suitable for brewing target alcoholic beverages to control expression level of the gene. Thus, alcoholic beverages can be produced highly-efficiently. That is to say, desired kind of alcoholic beverages can be produced highly-efficiently by controlling (elevating or reducing) flocculation property using yeasts into which the vector or DNA fragment of the present disclosure was introduced described above, yeasts in which expressions of the polynucleotide (DNA) of the present disclosure described above was regulated (promoted or suppressed) or yeasts selected by the yeast assessment method of the invention described below for fermentation to produce alcoholic beverages. The target alcoholic beverages include, for example, but not limited to beer, beer-taste beverages such as sparkling liquor (*happoushu*), wine, whisky, sake and the like. Further, alcohol for practical use such as alcohol for fuel is also included among them.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages. Thus, according to the present invention, alcoholic beverages can be produced highly-efficiently using the existing facility without increasing the cost.

## 5. Yeast assessment method of the invention

The present invention relates to a method for assessing a test yeast for its flocculation property by using a primer or a probe designed based on a nucleotide sequence of a gene having the nucleotide sequence of SEQ ID NO: 1 and encoding a protein imparting flocculation property to yeast. General techniques for such assessment method is known and is described in, for example, WO01/040514, Japanese Laid-Open Patent Application No. H8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene encoding a protein imparting flocculation property to yeast, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the flocculation property of yeast as determined by whether the molecular weight of the amplified product is a size that contains the DNA molecule of the specific part. In addition, by analyzing the nucleotide sequence of the amplified product, the property may be predicted and/or assessed more precisely.

Moreover, in the present invention, a test yeast is cultured to measure an expression level of the gene encoding a protein imparting flocculation property to yeast having the nucleotide sequence of SEQ ID NO: 1 to assess the test yeast for its flocculation property. In this case, the test yeast is cultured and then mRNA or a protein resulting from the gene encoding a protein imparting flocculation property to yeast is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the gene encoding a protein imparting flocculation property to yeast having the nucleotide sequence of SEQ ID NO:1 are measured to select a test yeast with the gene expression level according to the target flocculation property, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the gene having the nucleotide sequence of SEQ ID NO:1 is measured in each yeast. By selecting a test yeast with the gene expressed higher (i.e., expression level is enhanced) or lower (i.e., expression level is suppressed) than that in the reference yeast, a yeast suitable for brewing alcoholic beverages can be selected.

Alternatively, test yeasts are cultured and a yeast with a high or low flocculation property is selected, thereby selecting a yeast suitable for brewing desired alcoholic beverages.

In these cases, the test yeasts or the reference yeast may be, for example, a yeast introduced with the vector or DNA fragment of the invention, a yeast in which an expression of a polynucleotide (DNA) of the invention has been controlled, an artificially mutated yeast or a naturally mutated yeast. The flocculation property of yeast can be measured by, for example, a method described in Japanese Patent Application Laid-Open No. H8-205890. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g.,* Yasuji Oshima Ed., BIOCHEMISTRY EXPERIMENTS vol. 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (*e.g., S. pastorianus, S. cerevisiae,* and *S. carlsbergensis*). According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

## EXAMPLES

Hereinafter, the present invention will be described in more detail with reference to working examples. The present invention, however, is not limited to the examples described below.

### Example 1: Cloning of Gene Encoding Protein Responsible for Flocculation Property of Yeast (nonScHSP150)

A gene encoding a protein responsible for flocculation property of brewery yeast (nonScHSP150) (SEQ ID NO: 1) was found as a result of a search utilizing the comparison database described in Japanese Patent Application Laid-Open No. 2004-283169. Based on the acquired nucleotide sequence information, primers nonScHSP150_F (SEQ ID NO: 3) and nonScHSP150_R (SEQ ID NO: 4) were designed to amplify the full-length of the gene. PCR was carried out using chromosomal DNA of a genome sequencing strain, Saccharomyces pastorianus Weihenstephan 34/70 (sometimes abbreviated as "W34/79 strain"), as a template to obtain DNA fragments including the full-length gene of nonScHSP150.

The nonScHSP150 gene fragments thus obtained were inserted into pCR2.1-TOPO vector (Invitrogen) by TA cloning. The nucleotide sequences of the nonScHSP150 gene were analyzed by Sanger's method (F. Sanger, Science, 214: 1215, 1981) to confirm the nucleotide sequence.

### Example 2: Analysis of Expression of nonScHSP150 Gene during Beer Fermentation

A beer fermentation test was conducted using a lager brewing yeast, Saccharomyces pastorianus W34/70.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | $12.8 \times 10^6$ cells/mL |

The fermentation liquor was sampled over time, and the time-course changes in amount of yeast cell growth (Fig. 1) and apparent extract concentration (Fig. 2) were observed. Simultaneously, yeast cells were sampled to prepare mRNA, and the prepared mRNA was labeled with biotin and was hybridized to a beer yeast DNA microarray. The signal was detected using the GeneChip Operating system (GCOS; GeneChip Operating Software 1.0, manufactured by Affymetrix Co). Expression pattern of the nonScHSP150 gene is shown in Figure 3. This result confirmed the expression of the nonScHSP150 gene in the general beer fermentation.

**Example 3: Construction of nonScHSP150-Highly Expressed Strain**

The nonScHSP150/pCR2.1-TOPO described in Example 1 was digested with the restriction enzymes SacI and NotI to prepare a DNA fragment containing the entire length of the protein-encoding region. This fragment was ligated to pYCGPYNot treated with the restriction enzymes SacI and NotI, thereby constructing the nonScHSP150 high expression vector nonScHSP150/pYCGPYNot. pYCGPYNot is a YCp-type yeast expression vector. A gene inserted is highly expressed by the pyruvate kinase gene PYK1 promoter. The geneticin-resistant gene $G418^r$ is included as the selectable marker in the yeast, and the ampicillin-resistant gene $Amp^r$ as the selectable marker in Escherichia coli.

Using the high expression vector prepared by the above method, the strain Saccharomyces pasteurianus Weihenstephaner 34/70 was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

**Example 4: Evaluation of Flocculation Property of non-ScHSP150 Highly Expressed Strain**

Flocculation properties of the highly expressed strain obtained in Example 3 and the parent strain (W34/70 strain) were evaluated by a method described below.

The yeasts were inoculated into 50 mL of glucose CM medium (3% glucose, 1% bacto peptone, 0.5% yeast extract, 0.5% $KH_2PO_4$, 0.2% $MgSO_4.7H_2O$), then cultured statically at 30°C for 2 days.

Whole amount of the culture was transferred to 450 mL of glucose CM medium and cultured statically at 15°C for 4 days. The yeast cells were collected by centrifugation followed washing twice in cold water of 5°C. One point five grams (1.5 g) of the obtained wet yeast cells were accurately weighed, suspended in 20 mL of 0.1M acetate buffer containing 2mM Ca, and temperature equilibrated in an temperature controlled room at 15°C.

Subsequent measurement procedures were performed in a temperature controlled room at 15°C. The yeast suspension mixed for 30 seconds by vortex mixer was poured into a buret, then immediately 2 mL of the suspension was collected as a 0 min sample. After leaving 10 min at rest, 2 mL of the suspension was collected as a 10 min sample.

OD600 of the collected yeast suspensions were measured, respectively, and flocculation properties (Segmentation Index; SI) were calculated by formula below. The results are indicated in Figure 4. The values were averages of measurement of n = 2.

$$SI=(OD_{10min}-OD_{0min})/OD_{0min}*100$$

As indicated in Figure 4, SI = 261 in the highly expressed strain shows that flocculation property of yeast was increased by high expression of non-ScHSP150 by comparison with SI = 208 in the parent strain.

**Example 5: Disruption of nonScHSP150 Gene**

Fragments for gene disruption were prepared by PCR using a plasmid containing a drug resistance marker (pAG25(nat1)) as a template in accordance with a method described in literature (Goldstein et al., Yeast, 15, 1541 (1999)). Primers consisting of nonScHSP150_delta_for (SEQ ID NO. 5) and nonScHSP 150_delta_rv (SEQ ID NO. 6) were used for the PCR primers.

A spore clone (W34/70-2) isolated from lager brewing yeast Saccharomyces pastorianus strain W34/70 was transformed with the fragments for gene disruption prepared as described above. Transformation was carried out according to the method described in Japanese Patent Application Laid-open No. H07-303475, and transformants were selected on YPD plate medium (1% yeast extract, 2% polypeptone, 2% glucose, 2% agar) containing 50 mg/L of nourseothricin.

**Example 6: Evaluation of Flocculation Property of non-ScHSP150 Disrupted Strain**

Flocculation properties of the disrupted strain obtained in Example 5 and the parent strain (W34/70-2 strain) were evaluated by the same method as Example 4. As indicated in Figure 5, SI = 125 in the disrupted strain shows that flocculation property of yeast was decreased by disruption of non-ScHSP 150 by comparison with SI = 244 in the parent strain.

## INDUSTRIAL APPLICABILITY

The method of producing alcoholic beverages of the present invention may allow for highly-efficient production of alcoholic beverages by using a yeast having suitable for production of desired alcoholic beverages, because the flocculation property of yeast during fermentation can be controlled.

## Claims

1. A protein having an activity of imparting flocculation property to yeast encoded by a polynucleotide selected from the group consisting of

(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 in which one to 10 amino acids thereof are deleted, substituted, inserted and/or added, and having an activity of imparting flocculation property to yeast;
(d) a polynucleotide comprising a polynucleotide encoding a protein having an amino acid sequence having 95% or higher identity with the amino acid sequence of SEQ ID NO:2, and said protein having an activity of imparting flocculation property to yeast;
(e) a polynucleotide comprising a polynucleotide encoding a protein consisting of the amino acid sequence of SEQ ID NO: 2, or encoding the amino acid sequence of SEQ ID NO: 2 in which 1 to 5 amino acids thereof are deleted, substituted, inserted, and/or added, and wherein said protein has an activity of imparting flocculation property to yeast; and
(f) a polynucleotide comprising a polynucleotide encoding a protein having 98% or higher identity with the amino acid sequence of SEQ ID NO: 2, and having an activity of imparting flocculation property to yeast.

2. A yeast into which a vector containing a polynucleotide as defined in Claim 1 has been introduced, wherein the flocculation property is increased.

3. The yeast according to Claim 2, wherein the flocculation property is increased by increasing an expression level of the protein of Claim 1.

4. A yeast, wherein the expression of a polynucleotide as defined in Claim 1, is repressed by disrupting the gene according to the polynucleotide (DNA) as defined in Claim 1, said polynucleotide being a DNA.

5. The yeast according to Claim 4, wherein the flocculation property is decreased.

6. A method for producing an alcoholic beverage by using the yeast according to any one of Claims 2 to 5.

7. The method according to Claim 6, wherein the brewed alcoholic beverage is a malt beverage.

8. The method according to Claim 6, wherein the brewed alcoholic beverage is wine.

9. A method for assessing a test yeast for its flocculation property, comprising using a primer or probe designed based on the nucleotide sequence of a gene having the nucleotide sequence of SEQ ID NO: 1 and encoding a protein having an activity of imparting flocculation property to yeast.

10. A method for assessing a test yeast for its flocculation property, comprising: culturing the test yeast; and measuring the expression level of the gene having the nucleotide sequence of SEQ ID NO: 1 and encoding a protein having an activity of imparting flocculation property to yeast.

11. A method for selecting a yeast, comprising: culturing test yeasts; quantifying the protein of Claim 1 or measuring the expression level of the gene having the nucleotide sequence of SEQ ID NO: 1 and encoding a protein having an activity of imparting flocculation property to yeast; and selecting a test yeast having an amount of the protein or the gene expression level according to the flocculation property of interest.

12. The method for selecting a yeast according to Claim 11, comprising: culturing a reference yeast and test yeasts; measuring for each yeast the expression level of the gene having the nucleotide sequence of SEQ ID NO: 1 and encoding a protein having an activity of imparting flocculation property to yeast; and selecting a test yeast having the gene expression higher or lower than that in the reference yeast.

13. The method for selecting a yeast according to Claim 11, comprising: culturing a reference yeast and test yeasts; quantifying the protein according to Claim 1 in each yeast; and selecting a test yeast having a larger or smaller amount of the protein than that in the reference yeast.

14. A method for producing an alcoholic beverage comprising: conducting fermentation for producing an alcoholic beverage using the yeast according to any one of Claims 2 to 5.

15. Use of a polynucleotide as defined in Claim 1 for the preparation of a yeast with an increased flocculation property.

16. Use of a polynucleotide as defined in Claim 1 for controlling the flocculation property in yeast.

17. A method for controlling the flocculation property in a yeast comprising the step of controlling the expression of a polynucleotide as defined in Claim 1 in said yeast.

18. A method for increasing the flocculation property in a yeast comprising the step of increasing the expression of a polynucleotide as defined in Claim 1 in said yeast.

19. A method for decreasing the flocculation property in a yeast comprising the step of repressing the expression of a polynucleotide as defined in Claim 1 in said yeast.

**Patentansprüche**

1. Protein mit der Aktivität, einer Hefe Bruchbildungseigenschaft zu verleihen, codiert von einem Polynucleotid ausgewählt aus der Gruppe bestehend aus

(a) einem Polynucleotid umfassend ein Polynucleotid, das aus der Nucleotidsequenz von SEQ ID NO:1 besteht;

(b) einem Polynucleotid umfassend ein Polynucleotid, das ein Protein codiert, das aus der Aminosäuresequenz von SEQ ID NO:2 besteht;
(c) einem Polynucleotid umfassend ein Polynucleotid, das ein Protein codiert, das aus der Aminosäuresequenz von SEQ ID NO:2 besteht, in der eine bis 10 Aminosäuren davon entfernt, ersetzt, eingefügt, und/oder hinzugefügt sind, und wobei das Protein die Aktivität hat, einer Hefe Bruchbildungseigenschaft zu verleihen;
(d) einem Polynucleotid umfassend ein Polynucleotid, das ein Protein codiert, das eine Aminosäuresequenz hat, die eine 95%ige oder höhere Identität mit der Aminosäuresequenz von SEQ ID NO:2 hat, und wobei das Protein die Aktivität hat, einer Hefe Bruchbildungseigenschaft zu verleihen;
(e) einem Polynucleotid umfassend ein Polynucleotid, das ein Protein codiert, das aus der Aminosäuresequenz von SEQ ID NO:2 besteht, oder die Aminosäuresequenz von SEQ ID NO:2 codiert, in der 1 bis 5 Aminosäuren davon entfernt, ersetzt, eingefügt, und/oder hinzugefügt sind, und wobei das Protein die Aktivität hat, einer Hefe Bruchbildungseigenschaft zu verleihen; und
(f) einem Polynucleotid umfassend ein Polynucleotid, das ein Protein codiert, das eine 98%ige oder höhere Identität mit der Aminosäuresequenz von SEQ ID NO:2 hat, und wobei das Protein die Aktivität hat, einer Hefe Bruchbildungseigenschaft zu verleihen.

2. Hefe, in die ein Vektor enthaltend ein Polynucleotid wie in Anspruch 1 definiert eingeführt wurde, wobei die Bruchbildungseigenschaft gesteigert wird.

3. Hefe gemäß Anspruch 2, wobei die Bruchbildungseigenschaft gesteigert wird durch Steigern des Expressionspiegels des Proteins gemäß Anspruch 1.

4. Hefe, in der die Expression eines wie in Anspruch 1 definierten Polynucleotids durch die Unterbrechung des Gens gemäß dem wie in Anspruch 1 definierten Polynucleotid (DNA) unterdrückt ist, wobei das Polynucleotid DNA ist.

5. Hefe gemäß Anspruch 4, wobei die Bruchbildungseigenschaft herabgesetzt ist.

6. Verfahren zur Herstellung eines alkoholischen Getränks durch die Verwendung der Hefe gemäß einem der Ansprüche 2 bis 5.

7. Verfahren gemäß Anspruch 6, wobei das gebraute alkoholische Getränk ein Malzgetränk ist.

8. Verfahren gemäß Anspruch 6, wobei das gebraute alkoholische Getränk Wein ist.

9. Verfahren zur Bewertung der Bruchbildungseigenschaft einer Testhefe, umfassend die Verwendung eines Primers oder einer Sonde, der/die entwickelt wurde basierend auf der Nucleotidsequenz eines Gens, das die Nucleotidsequenz von SEQ ID NO:1 hat und das ein Protein codiert, das die Aktivität hat, einer Hefe Bruchbildungseigenschaft zu verleihen.

10. Verfahren zur Bewertung der Bruchbildungseigenschaft einer Testhefe, umfassend: Züchten der Testhefe; und Bestimmen des Expressionsspiegels des Gens, das die Nucleotidsequenz von SEQ ID NO:1 hat und das ein Protein codiert, das die Aktivität hat, einer Hefe Bruchbildungseigenschaft zu verleihen.

11. Verfahren zum Auswählen einer Hefe, umfassend: Züchten von Testhefen, Quantifizieren des Proteins gemäß Anspruch 1 oder Bestimmen des Expressionsspiegels des Gens, das die Nucleotidsequenz von SEQ ID NO:1 hat und das ein Protein codiert, das die Aktivität hat, einer Hefe Bruchbildungseigenschaft zu verleihen; und Auswählen einer Testhefe, die eine Proteinmenge oder den Genexpressionsspiegel entsprechend der Bruchbildungseigenschaft von Interesse hat.

12. Verfahren zum Auswählen einer Hefe gemäß Anspruch 11, umfassend: Züchten einer Referenzhefe und von Testhefen; Bestimmen des Expressionsspiegels des Gens, das die Nucleotidsequenz von SEQ ID NO:1 hat und das ein Protein codiert, das die Aktivität hat, einer Hefe Bruchbildungseigenschaft zu verleihen, in jeder Hefe; und Auswählen einer Testhefe, die das Gen stärker oder weniger als in der Referenzhefe exprimiert.

13. Verfahren zum Auswählen einer Hefe gemäß Anspruch 11, umfassend: Züchten einer Referenzhefe und von Testhefen; Quantifizieren des Proteins gemäß Anspruch 1 in jeder Hefe; und Auswählen einer Testhefe, die das Protein in einer größeren oder geringeren Menge hat als die Referenzhefe.

**14.** Verfahren zum Herstellen eines alkoholischen Getränks, umfassend: Durchführen einer Fermentation zur Herstellung eines alkoholischen Getränks unter Verwendung der Hefe gemäß einem der Ansprüche 2 bis 5.

**15.** Verwendung eines Polynucleotids wie in Anspruch 1 definiert für die Herstellung einer Hefe mit einer gesteigerten Bruchbildungseigenschaft.

**16.** Verwendung eines Polynucleotids wie in Anspruch 1 definiert zur Steuerung der Bruchbildungseigenschaft in Hefen.

**17.** Verfahren zum Steuern der Bruchbildungseigenschaft in einer Hefe umfassend den Schritt der Steuerung der Expression eines wie in Anspruch 1 definierten Polynucleotids in der Hefe.

**18.** Verfahren zum Steigern der Bruchbildungseigenschaft in einer Hefe umfassend den Schritt der Steigerung der Expression eines wie in Anspruch 1 definierten Polynucleotids in der Hefe.

**19.** Verfahren zum Herabsetzen der Bruchbildungseigenschaft in einer Hefe umfassend den Schritt der Repression der Expression eines wie in Anspruch 1 definierten Polynucleotids in der Hefe.

## Revendications

**1.** Protéine ayant une activité capable de conférer une propriété de floculation à la levure, codée par un polynucléotide choisi dans le groupe constitué par :

(a) un polynucléotide comprenant un polynucléotide se composant de la séquence de nucléotides de SEQ ID N° 1 ;
(b) un polynucléotide comprenant un polynucléotide codant une protéine se composant de la séquence d'acides aminés de SEQ ID N° 2 ;
(c) un polynucléotide comprenant un polynucléotide codant une protéine se composant de la séquence d'acides aminés de SEQ ID N° 2, dans laquelle un à 10 acides aminés de celle-ci sont supprimés, substitués, insérés et/ou ajoutés, et ayant une activité capable de conférer une propriété de floculation à la levure ;
(d) un polynucléotide comprenant un polynucléotide codant une protéine ayant une séquence d'acides aminés présentant une identité de 95 % ou plus avec la séquence d'acides aminés de SEQ ID N° 2, et ladite protéine ayant une activité capable de conférer une propriété de floculation à la levure ;
(e) un polynucléotide comprenant un polynucléotide codant une protéine se composant de la séquence d'acides aminés de SEQ ID N° 2, ou codant la séquence d'acides aminés de SEQ ID N° 2, dans laquelle 1 à 5 acides aminés de celle-ci sont supprimés, substitués, insérés et/ou ajoutés, et où ladite protéine a une activité capable de conférer une propriété de floculation à la levure ; et
(f) un polynucléotide comprenant un polynucléotide codant une protéine présentant une identité de 98 % ou plus avec la séquence d'acides aminés de SEQ ID N° 2, et ayant une activité capable de conférer une propriété de floculation à la levure.

**2.** Levure, dans laquelle a été introduit un vecteur contenant un polynucléotide tel que défini dans la revendication 1, où la propriété de floculation est accrue.

**3.** Levure selon la revendication 2, dans laquelle la propriété de floculation est accrue grâce à l'accroissement d'un taux d'expression de la protéine selon la revendication 1.

**4.** Levure, dans laquelle l'expression d'un polynucléotide tel que défini dans la revendication 1 est réprimée par interruption du gène selon le polynucléotide (ADN) tel que défini dans la revendication 1, ledit polynucléotide étant un ADN.

**5.** Levure selon la revendication 4, dans laquelle la propriété de floculation est diminuée.

**6.** Procédé de production d'une boisson alcoolique en utilisant la levure selon l'une quelconque des revendications 2 à 5.

**7.** Procédé selon la revendication 6, dans lequel la boisson alcoolique brassée est une boisson maltée.

**8.** Procédé selon la revendication 6, dans lequel la boisson alcoolique brassée est le vin.

**9.** Procédé d'évaluation d'une levure test pour sa propriété de floculation, comprenant l'utilisation d'une amorce ou d'une sonde conçue sur la base de la séquence de nucléotides d'un gène ayant la séquence de nucléotides de SEQ ID N° 1 et le codage d'une protéine ayant une activité capable de conférer une propriété de floculation à la levure.

**10.** Procédé d'évaluation d'une levure test pour sa propriété de floculation, comprenant : la culture de la levure test ; et la mesure du taux d'expression du gène ayant la séquence de nucléotides de SEQ ID N° 1 et le codage d'une protéine ayant une activité capable de conférer une propriété de floculation à la levure.

**11.** Procédé de sélection d'une levure, comprenant : la culture de levures tests ; la quantification de la protéine selon la revendication 1 ou la mesure du taux d'expression du gène ayant la séquence de nucléotides de SEQ ID N° 1 et le codage d'une protéine ayant une activité capable de conférer une propriété de floculation à la levure ; et la sélection d'une levure test ayant une quantité de protéine ou le taux d'expression du gène correspondant à la propriété de floculation d'intérêt.

**12.** Procédé de sélection d'une levure selon la revendication 11, comprenant: la culture d'une levure de référence et de levures tests ; la mesure du taux d'expression du gène ayant la séquence de nucléotides de SEQ ID N° 1 pour chaque levure et le codage d'une protéine ayant une activité capable de conférer une propriété de floculation à la levure ; et la sélection d'une levure test ayant un taux d'expression du gène plus élevé ou plus faible que la levure de référence.

**13.** Procédé de sélection d'une levure selon la revendication 11, comprenant: la culture d'une levure de référence et de levures tests ; la quantification de la protéine selon la revendication 1 dans chaque levure ; et la sélection d'une levure test ayant une quantité de protéine supérieure ou inférieure à celle de la levure de référence.

**14.** Procédé de production d'une boisson alcoolique comprenant : la réalisation de la fermentation pour la production d'une boisson alcoolique en utilisant la levure selon l'une quelconque des revendications 2 à 5.

**15.** Utilisation d'un polynucléotide tel que défini dans la revendication 1 pour la préparation d'une levure ayant une propriété de floculation accrue.

**16.** Utilisation d'un polynucléotide tel que défini dans la revendication 1 pour le contrôle de la propriété de floculation dans la levure.

**17.** Procédé de contrôle de la propriété de floculation dans une levure comprenant l'étape de contrôle de l'expression d'un polynucléotide tel que défini dans la revendication 1 dans ladite levure.

**18.** Procédé d'augmentation de la propriété de floculation dans une levure comprenant l'étape d'accroissement de l'expression d'un polynucléotide tel que défini dans la revendication 1 dans ladite levure.

**19.** Procédé de diminution de la propriété de floculation dans une levure comprenant l'étape de répression de l'expression d'un polynucléotide tel que défini dans la revendication 1 dans ladite levure.

FIG. 1

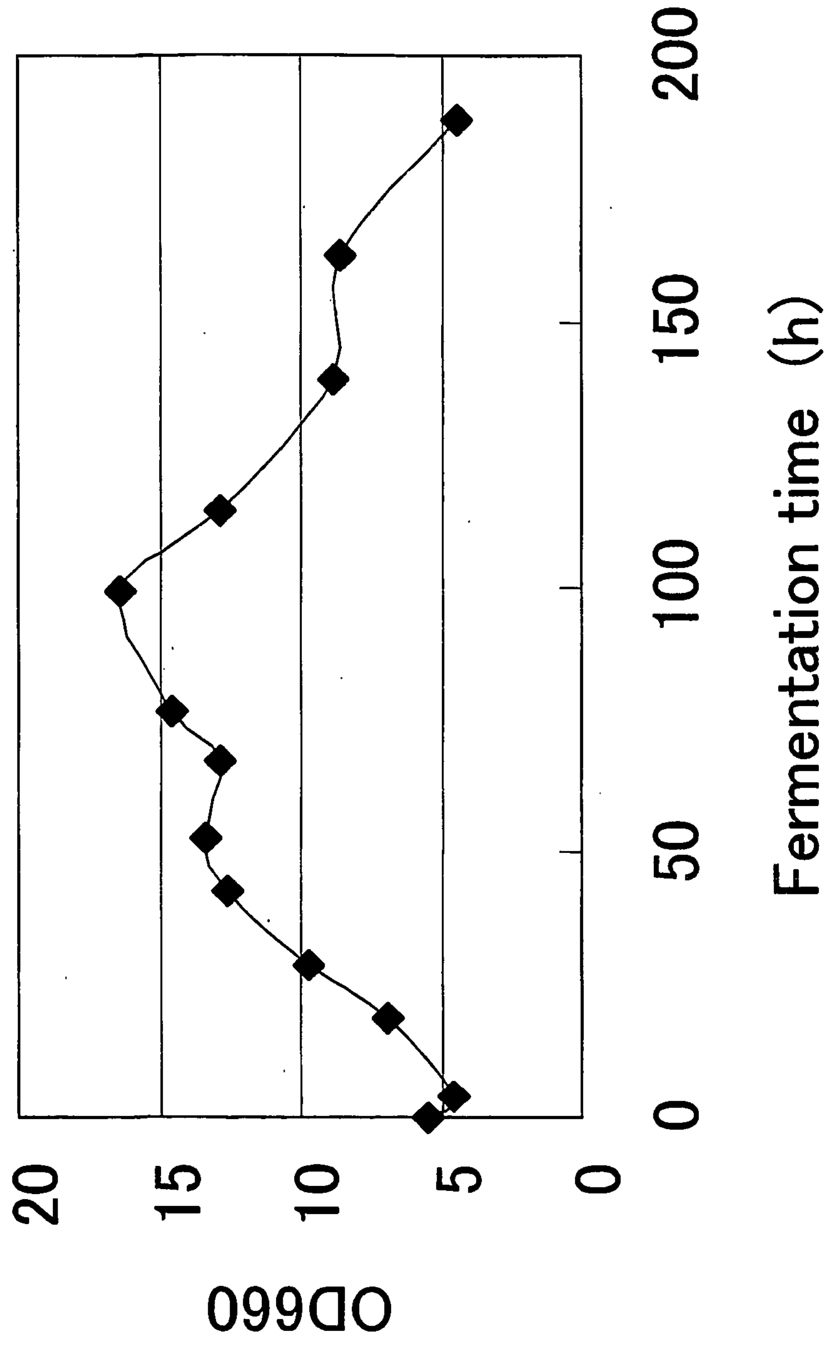

FIG. 2

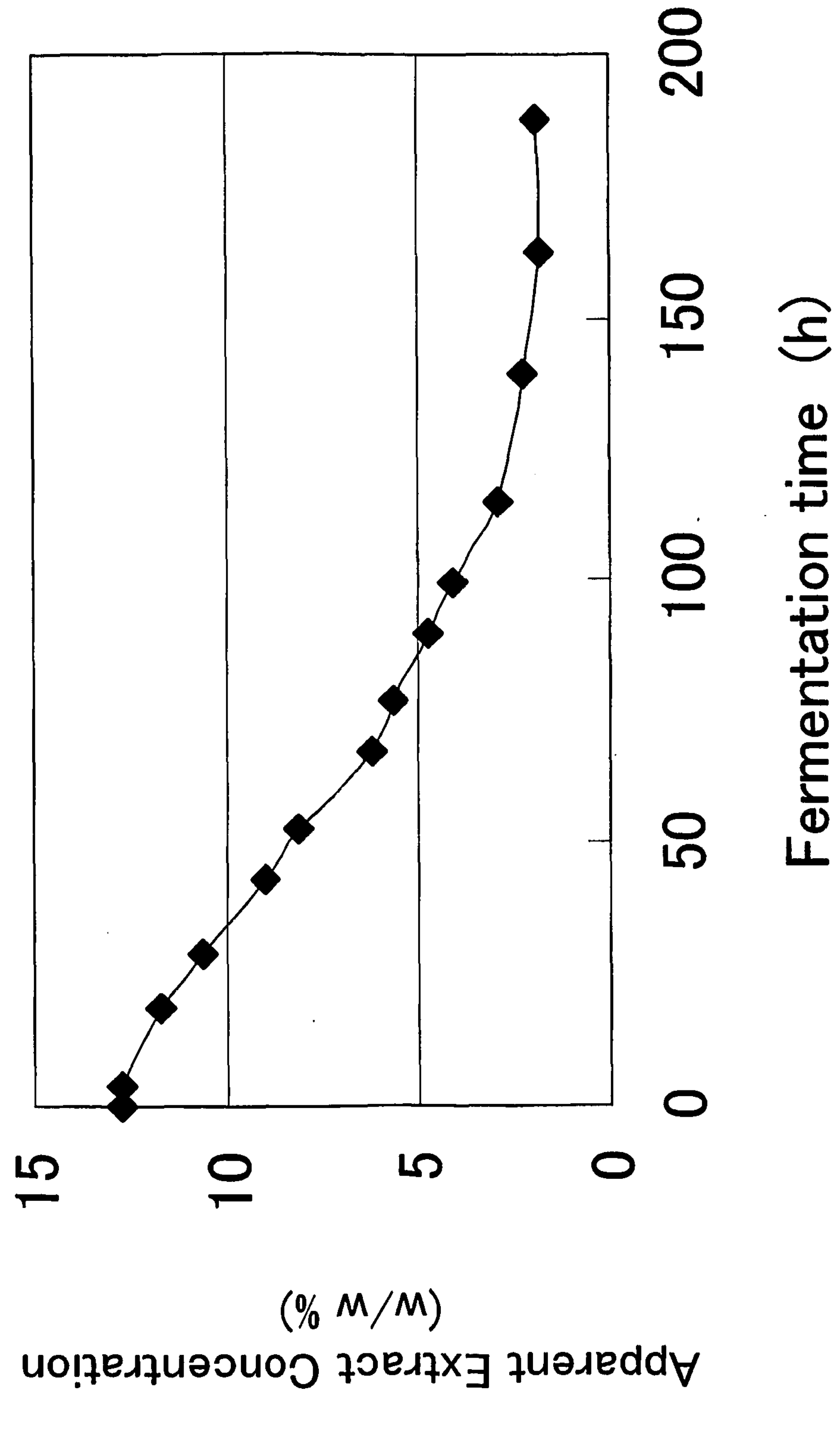
Apparent Extract Concentration (w/w %)
15
10
5
0
0
50
100
150
200
Fermentation time (h)

FIG. 3

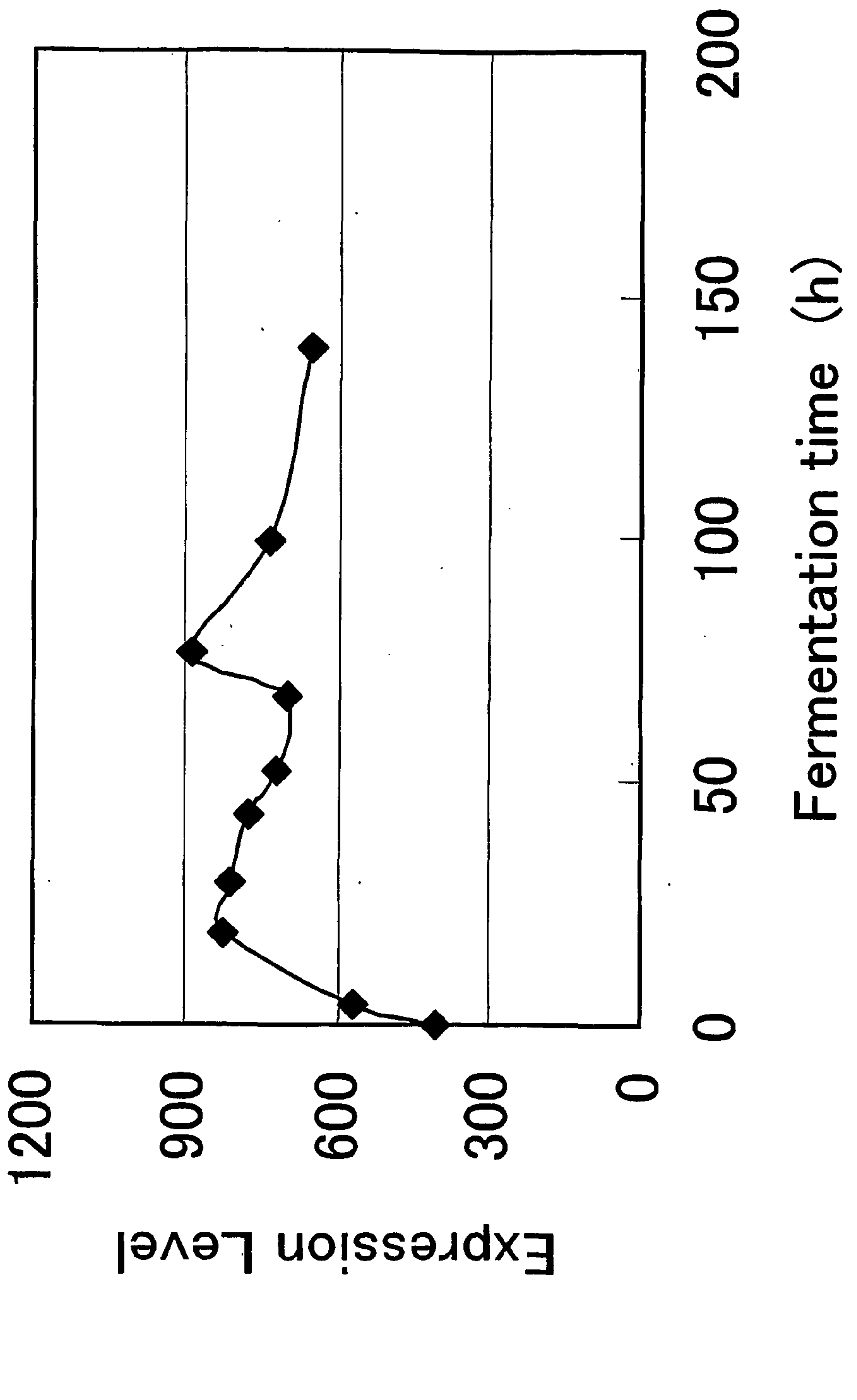
Expression Level
1200
900
600
300
0
0
50
100
150
200
Fermentation time (h)

FIG. 4

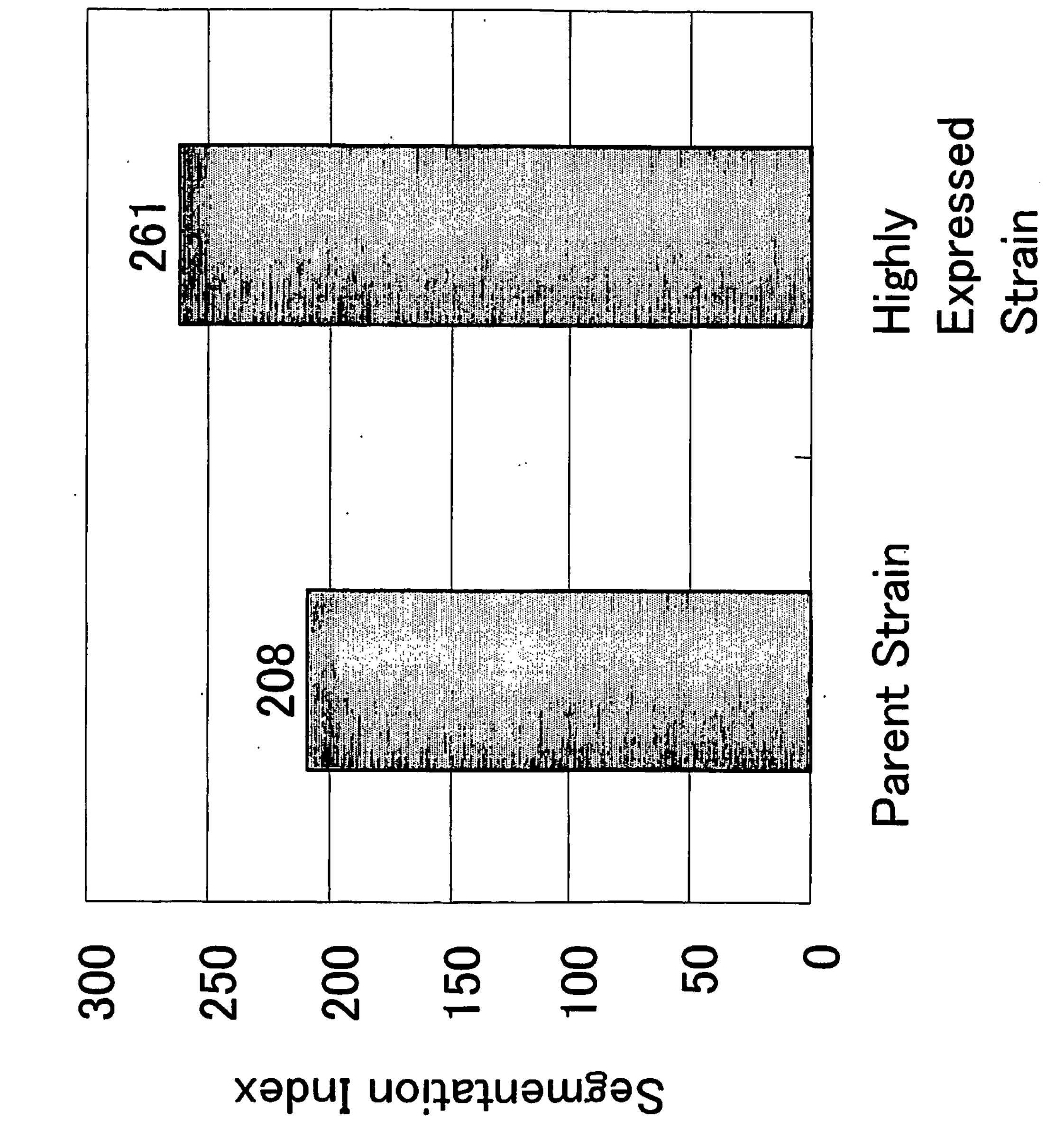
Segmentation Index
300
250
200
150
100
50
0
208
261
Parent Strain
Highly Expressed Strain

FIG. 5

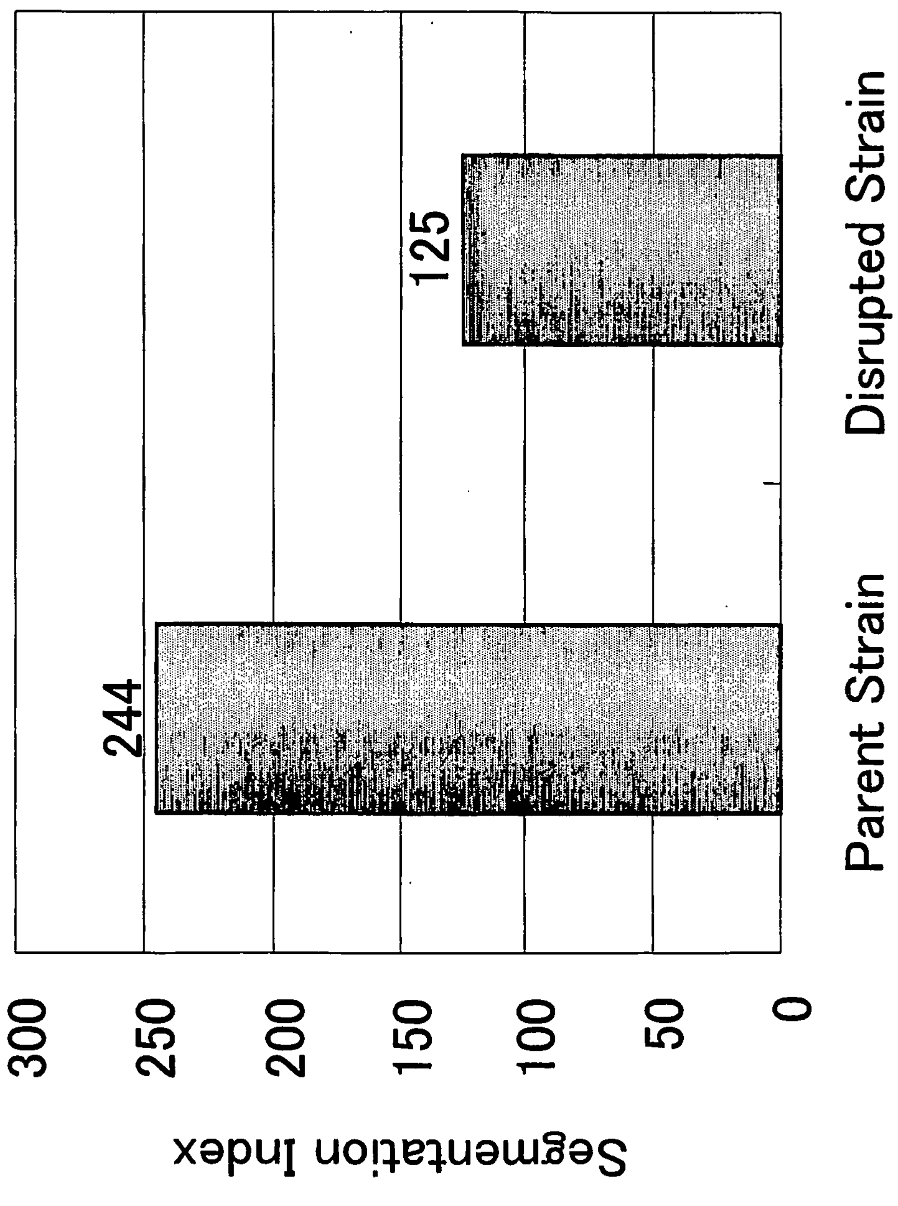
Segmentation Index
300
250
200
150
100
50
0
244
125
Parent Strain
Disrupted Strain

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3044284 B
- JP 3643404 B
- WO 01040514 A
- JP 2004383169 A
- JP H8205890 B
- JP 2002516062 PCT
- US 2002086356 A
- JP 6253826 A
- JP H8205900 B
- JP 2004283169 A
- JP H07303475 B
- JP 2006049223 A


### Non-patent literature cited in the description


- **Bidard et al.** *YEAST,* 1995, vol. 11 (9), 809
- MOLECULAR CLONING. CURRENT PROTOCOLS IN MOLECULAR BIOLOGY. John Wiley & Sons, 1987
- **Karlih ; Altschul.** *Proc. Natl. Acad Sci. USA,* 1990, vol. 87, 2264-2268
- *Proc. Natl. Acad. Sci. US4,* 1993, vol. 90, 5873
- **Altschul SF et al.** *J. Mol. Biol.,* 1990, vol. 215, 403
- **Hirajima ; Inoue.** New Biochemistry Experiment Course 2 Nucleic Acids IV Gene Replication and Expression. Tokyo Kagaku Dozin Co., Ltd, 1993, 319-347
- *Nature Genetics,* February 2000, vol. 24 (2), 180-183
- *Genesis,* April 2000, vol. 26 (4), 240-244
- *Nature,* 21 September 2002, vol. 407 (6802), 319-20
- *Genes & Dev.,* 15 April 2002, vol. 16 (8), 948-958
- *Proc. Natl. Acad. Sci. USA.,* 16 April 2002, vol. 99 (8), 5515-5520
- *Science,* 19 April 2002, vol. 296 (5567), 550-553
- *Proc Natl. Acad. Sci. USA,* 30 April 2002, vol. 99 (9), 6047-6052
- *Nature Biotechnology,* May 2002, vol. 20 (5), 497-500
- *Nature Biotechnology,* May 2002, vol. 20 (5), 500-505
- *Nucleic Acids Res.,* 15 May 2002, vol. 30 (10), e46
- *FEBS Lett.,* 1988, vol. 228, 228
- *FEBS Lett.,* 1988, vol. 239, 285
- *Nucl. Acids. Res.,* 1989, vol. 17, 7059
- *Nature,* 1986, vol. 323, 349
- *Nucl. Acids. Res.,* 1991, vol. 19, 6751
- *Protein Eng,* 1990, vol. 3, 733
- **NUCL. ACIDS RES.** *Nucl. Acids Res.,* 1991, vol. 19, 3875
- *Nucl. Acids Res.,* 1991, vol. 19, 5125
- *Biochem Biophys Res Commun,* 1992, vol. 186, 1271
- **Smyth DR.** *Curr. Biol.,* 1997, vol. 7, R793
- **Martienssen R.** *Curr. Biol,* 1996, vol. 6, 810
- MOLECULAR CLONING. CURRENT PROTOCOLS IN MOLECULAR BIOLOGY
- *Nuc. Acids. Res.,* 1982, vol. 10, 6487
- *Proc. Natl. Acad Sci. USA,* 1982, vol. 79, 6409
- *Gene,* 1985, vol. 34, 315
- *Nuc. Acids. Res.,* 1985, vol. 13, 4431
- *Proc. Natl. Acad Sci. USA,* 1985, vol. 82, 488
- *Proc. Natl. Acad. Sci. USA,* 1979, vol. 76, 4951
- *Methods in Enzymology,* 1983, vol. 101, 202
- *Nucleic Acids Res.,* 2001, vol. 29, 4238-4250
- *Appl Environ Microbiol.,* 2006, vol. 72, 5266-5273
- **J. R Broach et al.** EXPERIMENTAL MANIPULATION OF GENE EXPRESSION. Academic Press, 1983, vol. 83
- **M. D. Rose et al.** *Gene,* 1987, vol. 60, 237
- **K. Struhl et al.** *Proc. Natl. Acad Sci. USA,* 1979, vol. 76, 1035
- **M. F. Tuite et al.** *EMO J.,* 1982, vol. 1, 603
- **Marin et al.** *Proc. Natl. Acad. Sci. USA,* 1984, vol. 81, 337
- **Junji Inokoshi et al.** *Biochemistry,* 1992, vol. 64, 660
- **Hussain et al.** *Gene,* 1991, vol. 101, 149
- *Meth. Enzym.,* 1990, vol. 194, 182
- *Proc. Natl. Acad Sci. USA,* 1978, vol. 75, 1929
- *J. Bacteriology,* 1983, vol. 153, 163
- METHODS IN YEAST GENETICS. Course Manual. A Cold Spring Harbor Laboratory, 2000
- Genetic Engineering. lenum Press, 1979, vol. 1, 117

- MOLECULAR CLONING. METHODS IN YEAST GENETICS, A LABORATORY MANUAL. Cold Spring Harbor Laboratory Press
- METHODS IN YEAST GENETICS. Cold Spring Harbor Laboratory Press, 1990, vol. 130
- CURRENT PROTOCOLS IN MOLECULAR BIOLOGY. John Wiley & Sons, 1994
- Yeast Molecular Genetic Experiments. BIOCHEMISTRY EXPERIMENTS. vol. 39, 67-75
- **F. Sanger.** *Science,* 1981, vol. 214, 1215
- **Goldstein et al.** *Yeast,* 1999, vol. 15, 1541